# EUROPEAN PATENT SPECIFICATION

(11) **EP 2 852 375 B1**
(45) Date of publication and mention of the grant of the patent: **04.04.2018**
(21) Application number: 13715708.7
(22) Date of filing: 15.04.2013
(51) Int. Cl.: A61K 8/365, A61Q 5/04

(54) **METHOD OF TREATING HAIR**
HAARBEHANDLUNGSVERFAHREN
PROCÉDÉ DE TRAITEMENT DES CHEVEUX

(30) Priority: 21.05.2012 EP 12168631; 04.03.2013 EP 13157665
(43) Date of publication of application: 01.04.2015
(73) Proprietor: Unilever PLC, London EC4Y 0DY (GB); Unilever N.V., 3013 AL Rotterdam (NL)
(72) Inventor: MURRAY, Andrew, Malcolm, Bebington Wirral Merseyside CH63 3JW (GB); PAUL, Prem, Kumar, Cheyalazhagan, Bebington Wirral Merseyside CH63 3JW (GB)
(74) Representative: James, Helen Sarah
(86) International application number: PCT/EP2013/057809
(87) International publication number: WO 2013/174575

(56) References cited:
- EP-A1- 1 393 708
- WO-A1-03/039497
- WO-A1-2005/025524
- WO-A1-2010/141098
- WO-A2-2010/049434
- WO-A2-2012/084532
- US-A- 4 349 537

## Description

The invention relates to a method of hair straightening.

Permanent hair straightening compositions that are on the market are based on chemical treatment of the hair in a two-step process using thiol- or hydroxide-based reducing agents followed by a neutralisation or oxidation step. Such systems have various negatives associated with them; in that the process itself is difficult to conduct, in many instances this straightening process is undertaken by a qualified hairdresser in a professional salon. Furthermore the straightening process damages the hair, has an unpleasant odor and can cause irritation to the scalp.

WO2010049434 discloses a process for straightening or relaxing hair using a composition having a pH of from 8 to 11.5 and comprising at least one weak acid chosen from monocarboxylic, dicarboxylic and tricarboxylic acids. WO2005025524 discloses a hair straightening composition comprising reducing agents and alpha-hydroxy acids.

WO2010141098 discloses a one-part solution for straightening or curling hair comprising several hydrolysed proteins and other components (claim 1). The solution may also comprise 0.1 % citric acid.

WO03039497 discloses the use of an acidic clarifying treatment after a hair straightening treatment.

US4349537) discloses a permanent waving neutraliser and oxidising solution comprising citrate ion and citric acid to provide a pH of 1.9 to 4, and an oxidising agent.

EP1393708 discloses a process for straightening hair which comprises applying a first pack comprising a keratin-reducing substance, then applying a second pack with an organic acid.

The present invention has now found that hair can be straightened in a way that mitigates damage; the hair remains straight even after subsequent washing.

### Summary of Invention

The present invention relates to a process of treating the hair comprising the following consecutive steps:
i) applying to dry hair a hair treatment composition comprising at least 4wt% of the total composition of a bidentate or tridentate carboxylic acid having a pH of 3 or less at 20°C
ii) leaving the product on the hair for at least 5 minutes;
iii) rinsing the product from the hair; and
iv) combing,

in which the carboxylic acid is tartaric acid and/or citric acid.

### Description of Invention

This invention is advantageous if used for hair straightening.

Compositions of the invention comprise a bidentate or tridentate carboxylic acid which is tartaric acid and/or citric acid, citric acid is particularly preferred.

The total level of bidentate or tridentate of carboxylic acid is greater than 4 wt of the total composition, more preferably 5 wt% of greater most preferably 8 wt% or greater.

Hair care compositions of the present invention can comprise a carrier, or a mixture of such carriers, which are suitable for application to the hair. The carriers are present at from about 0.5% to about 99.5%, preferably from about 5.0% to about 99.5%, more preferably from about 10.0% to about 98.0%, of the composition. As used herein, the phrase "suitable for application to hair" means that the carrier does not damage or negatively affect the aesthetics of hair or cause irritation to the underlying skin.

Compositions according to the invention comprise a buffer or pH adjuster. Preferred buffers or pH adjusters include weak acids and bases such glycine/sodium hydroxide, lactic acid, succinic acid, acetic salt and salts thereof. Frequently a mixture of buffering systems is used.

Compositions according to the invention are preferably aqueous compositions, in some instances intended to be applied to the hair after shampooing and rinsing. The compositions are massaged into dry hair, left on the hair for at least 5 minutes (the hair may be heated) followed by further rinsing with water prior and combing. By aqueous composition, it is meant that the compositions of the invention comprise 60% by weight or more of water, preferably 70% or more, more preferably 80% or more.

When the hair care composition is a lotion, cream, tonic, gel, or mousse the preferred solvents include water, ethanol, volatile silicone derivatives, and mixtures thereof. The solvents used in such mixtures may be miscible or immiscible with each other. Mousses can also utilise any of the conventional propellants to deliver the material as a foam (in the case of a mousse). Examples of suitable propellants include materials such as trichlorofluoromethane, dichlorodifluoromethane, difluoroethane, dimethylether, propane, n-butane or isobutane. A tonic product having a low viscosity may also utilise an emulsifying agent. Examples of suitable emulsifying agents include nonionic, cationic, anionic surfactants, or mixtures thereof. If such an emulsifying agent is used, it is preferably present at a level of from about 0.01 % to about 7.5% by weight based on total weight of the composition. The level of propellant can be adjusted as desired but is generally from about 3% to about 30% by weight based on total weight for mousse compositions and from about 15% to about 50% by weight based on total weight for aerosol hair spray compositions.

Hair styling creams or gels also typically contain a structurant or thickener, typically in an amount of from 0.01 % to 10% by weight.

The formulation may include conditioning materials such as surfactants, cationic conditioners suitable for hair, quaternary silicone polymers, silicone based conditioners and their emulsions, and amino functional silicones and their emulsions.

Further general ingredients suitable for all product forms include: sun-screening agents, anti-dandruff actives, carboxylic acid polymer thickeners and emulsifiers for emulsifying the various carrier components of the compositions of the invention.

In some aspects of this invention it is highly desirable if the composition comprises a styling aid.

Particularly useful as styling aids with this invention are hair styling polymers. Hair styling polymers are well known articles of commerce and many such polymers are available commercially which contain moieties which render the polymers cationic, anionic, amphoteric or nonionic in nature. The polymers may be synthetic or naturally derived.

The amount of the hair styling polymer may range from 0.1 to 10%, preferably 0.5 to 8 %, more preferably 0.75 to 6% by weight based on total weight of the composition.

The compositions of the present invention may also contain adjuncts suitable for hair care. Generally such ingredients are included individually at a level of up to 2, preferably up to 1 wt% of the total composition. Suitable hair care adjuncts, include amino acids, sugars and ceramides.

Although the product may be in any form suitable for application to the hair it is preferable if it is a rinse off product. Products used to condition the hair are especially preferred.

In use the composition of the invention is applied to the hair and left on the hair for at least, 5 minutes, preferably at least 10 minutes, more preferably at least 15 minutes. Preferably the product is rinsed off 90 minutes after application, more preferably 60 minutes after application, most preferably this product is rinsed off 40 minutes after application.

The following non-limiting Examples further illustrate the preferred embodiments of the invention. All percentages referred to in the examples and throughout this specification are by weight based on total weight unless otherwise indicated.

### Examples

### Experiment 1

To demonstrate the straightening benefit of citric acid as a function of pH.

Dark brown European wavy#6 switches of length 25 cm and weight 2gms, were dosed with 2ml each of **5**% citric acid solutions at various pH's. They were combed straight and left to dry for at least 20 minutes. They were subsequently rinsed for 30 seconds under the tap. They were then combed straight and left to dry overnight. When dry the switches were combed straight and pictures taken. The volume of the switches shows the straightening benefit of citric acid. (Here volume refers to the projection of the switch image on to the screen and is given in mm²).

| **Treatment** | **Volume in mm^2** | **%benefit over water** |
|---|---|---|
| water | 14278 | 0 |
| 5% citric acid at pH 2 | 9481 | 33.6 |
| 5% citric acid at pH 3 | 9574 | 32.9 |
| 5% citric acid at pH 4 | 11717 | 17.9 |
| 5% citric acid at pH 5 | 10871 | 23.9 |
| 5% citric acid at pH 6 | 12136 | 15.0 |

From the table it can be seen that though there is benefit of citric acid solution up to pH 6, the benefits is quite marked at pH at 3 and below.

### Experiment 2:

In this experiment the effect of re-wetting some of the switches above is looked at. Some switches in the previous experiment were re-wetted, combed straight and left to dry. When dry the switches were combed and pictures taken.

| **Treatment** | **Volume in mm²** | **%benefit over water** |
|---|---|---|
| water | 14081 | 0 |
| 5% citric acid at pH 2 | 10205 | 27.5 |
| 5% citric acid at pH 4 | 11509 | 18.3 |

The table above shows that the straightness benefit is maintained even after re-wetting.

### Experiment 3:

5% citric acid at pH 2 and at pH 3 was applied to hair as described above, however the hair was wet. The straightening benefit was significantly reduced compared with the results with dry hair.

### Experiment 4:

Following the procedure outlined in Experiment 1, other di and tri carboxylic acids were looked at and the results are given in the next table. While dosing is the method of application, for actives that were not soluble at the 5% level (glutamic, fumaric, succinic and adipic) the switches were soaked in the solutions. Only those that showed a benefit above 15% at pH 2 were looked at higher pH's

| **Treatment** | **Volume in mm^2** | **%benefit over water** |
|---|---|---|
| water | 14278 | 0 |
| 5% Tartaric acid at pH 2 | 11146 | 21.9 |
| 5% Tartaric acid at pH 3 | 11859 | 16.9 |

## Claims

1. A process of treating the hair comprising the following consecutive steps:
i) applying to dry hair a hair treatment composition having a pH of 3 or less at 20°C and comprising at least 4 wt% of the total composition of a bidentate or tridentate carboxylic acid;
ii) leaving the product on the hair for at least 5 minutes;
iii) rinsing the product from the hair; and
iv) combing;
in which the carboxylic acid is tartaric acid and/or citric acid.

2. A process according to claim 1 in which the composition is left on the hair for at least 10 minutes.

3. A process according to claim 1 or claim 2 in which the carboxylic acid is citric acid.

4. A process according to any preceding claim in which the level of carboxylic acid is greater than 8 wt% of the total composition.

## Patentansprüche

1. Haarbehandlungsverfahren, umfassend die nachstehenden aufeinander folgenden Schritte:
i) Aufbringen einer Haarbehandlungszusammensetzung, die einen pH von 3 oder weniger bei 20°C aufweist und die mindestens 4 Gew.-% der gesamten Zusammensetzung an einer zwei- oder dreizähnigen Carbonsäure umfasst, auf trockenes Haar,
ii) Belassen des Produkts auf dem Haar für mindestens 5 Minuten,
iii Spülen des Produktes von dem Haar und
iv) Kämmen,
worin die Carbonsäure Weinsäure und/oder Zitronensäure darstellt.

2. Verfahren nach Anspruch 1, worin die Zusammensetzung für mindestens 10 Minuten auf dem Haar belassen wird.

3. Verfahren nach Anspruch 1 oder Anspruch 2, worin die Carbonsäure Zitronensäure darstellt.

4. Verfahren nach einem vorhergehenden Anspruch, worin der Anteil an Carbonsäure mehr als 8 Gew.-% der gesamten Zusammensetzung darstellt.

## Revendications

1. Procédé de traitement des cheveux comprenant les étapes consécutives suivantes consistant :
i) à appliquer aux cheveux secs une composition de traitement des cheveux ayant un pH de 3 ou inférieur à 20°C et comprenant au moins 4 % en masse de la composition totale d'un acide carboxylique bidenté ou tridenté ;
ii) à laisser le produit sur les cheveux pendant au moins 5 minutes ;
iii) à rincer le produit des cheveux ; et
iv) à peigner ;
dans lequel l'acide carboxylique est l'acide tartarique et/ou l'acide citrique.

2. Procédé selon la revendication 1, dans lequel la composition est laissée sur les cheveux pendant au moins 10 minutes.

3. Procédé selon la revendication 1 ou la revendication 2, dans lequel l'acide carboxylique est l'acide citrique.

4. Procédé selon l'une quelconque des revendications précédentes, dans lequel la teneur en acide carboxylique est supérieure à 8 % en masse de la composition totale.
